# EUROPEAN PATENT SPECIFICATION

(11) **EP 0 990 428 B1**
(45) Date of publication and mention of the grant of the patent: **07.07.2004**
(21) Application number: 99307699.1
(22) Date of filing: 29.09.1999
(51) Int. Cl.: A61F 2/46

(54) **Pressuriser device**
Vorrichtung zum Unterdrucksetzen
Dispositif de pressurisation

(30) Priority: 30.09.1998 GB 9821182
(43) Date of publication of application: 05.04.2000
(73) Proprietor: Johnson & Johnson Medical Limited, Ascot, Berkshire SK5 9EY (GB)
(72) Inventor: Naybour, John, Mold, Flintshire (GB); Revie, Ian Crawford, New Milton, Hampshire BH25 5BU (GB)
(74) Representative: Fisher, Adrian John

(56) References cited:
- EP-A- 0 320 138
- WO-A-90/00375
- US-A- 4 815 454
- US-A- 4 827 919
- US-A- 4 997 448
- US-A- 5 766 262

## Description

The present invention relates to a pressuriser device particularly for use in cemented femoral hip stem replacement. The pressuriser device is also suitable for use in other cemented prosthetic applications.

A femoral bone is prepared for a replacement hip stem prosthesis by expanding the internal cavity of the femoral bone by using a broach or rasp. The broached cavity is filled with bone cement either before or after the insertion of the hip stem prosthesis.

In the method in which a hip stem prosthesis is inserted after the cement is introduced into the broached cavity, it is important to centralise the stem within the prepared femoral cavity and to maintain the pressure of the cement surrounding the hip stem prosthesis. Centralisation of a hip stem prosthesis and pressurisation of the cement have been linked to long term stem survivability in hip replacement surgery.

Known pressuriser devices are either removed prior to stem introduction or a part of the pressuriser is removed or changed to a separate component thereby disturbing the cement and the pressure generation. It is an object of the present invention to maintain the pressure on the cement without any disturbance.

EP 0 320 138 discloses a proximal femoral sealing device for use in the application of bone cement under pressure to a surgically prepared medullay canal of a femur prior to the implantation of a hip prosthesis. WO 90/00375 discloses a device for sealing-off the medullay canal when bone cement is applied.

According to the present invention, there is provided a pressuriser device having a collar defining an opening for accommodating a prosthetic component, and a hood attached to the collar which can enclose at least a portion of the central opening of the collar.

The hood is moveable between two positions, a first position in which the hood covers the opening and a second position in which the opening is uncovered.

Preferably, the collar has an outer surface corresponding to the shape of a bone cavity in which the prosthetic component is to be inserted.

Preferably, the hood has a hoop extending across the opening in the collar. Preferably, a body of the hood extends from the hoop to the collar.

Preferably, the hoop conforms to the shape of the prosthetic component. The hoop may be attached to the collar by a sprung hinge against which it can fold laterally.

Optimally, the body of the hood corresponds to the shape of the prosthetic component to be inserted. The hood may be attached to the collar along the anterior and posterior aspects of the collar.

Preferably, the hood is attached by a tearable and flexible membrane.

Preferably, the hood is lined with an insert to stiffen the hood. The insert may also conform to the shape of the prosthetic component.

An embodiment of a pressuriser device in accordance with the present invention is now described, by means of example only, with reference to:

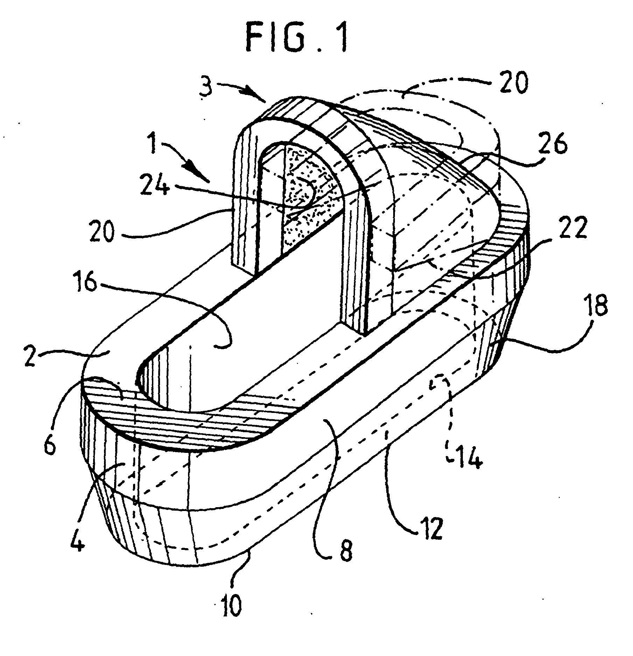

Figure 1 is a perspective view of a pressuriser device in accordance with the present invention.

Referring to the drawing, a pressuriser device 1 is provided which is formed in two parts, a collar 4 and a hood 3. The collar 4 is formed of a flexible material such as medical grade silicone and the hood 3 is formed from silicone with and insert of high density polyethylene. The body 2 of the pressuriser device 1 is in the form of a collar 4 with a top surface 6, sides 8 and a bottom surface 12.

The collar 4 defines a central opening 14. The central opening 14 of the collar 4 corresponds to the shape of the prosthetic component, in this example a hip stem. The opening 14 has internal walls 16 which fit the proximal section of a hip stem.

The body 2 has a lower section 10 with external walls 18. The external walls 18 of the lower section 10 correspond to a broached femoral canal in which the pressuriser device 1 is inserted such that the body 2 sits in the entry to the broached canal of the patient's bone.

The pressuriser device 1 has a hood 3 with a hoop 20 which extends in an arch across the opening 14 of the collar 4. As the opening 14 of the collar 4 corresponds to a proximal section of a hip stem, it is generally oval in shape and the hoop 20 extends centrally across the narrow width of the oval shape. The hoop 20 corresponds to the hip stem shape. A sprung hinge 22 attaches the hoop 20 to the collar 4. The hoop 20 folds laterally against the sprung hinge 22.

The hood 3 is lined with a polyethylene insert in the form of the hood lining 24 which assists in stem introduction by stiffening the hood 3 and allowing the stem to be introduced easily. The hood lining 24 also conforms to the shape of the hip stem.

The hood 3 is attached to the pressuriser device 1 along the anterior and posterior aspects of the top surface 6 of the collar 4 by a tearable and flexible membrane which is designed to rip or stretch on opening the hood 3.

The purpose of the present invention is to allow the introduction of PMMA bone cement into a broach femoral canal using pressurised syringe introduction. The pressuriser device 1 allows pressurisation of the cement in the femoral cavity.

The pressuriser device 1 also allows stem introduction without removal or exchange of any of the pressuriser components. Control of the final proximal positioning of the stem in the femoral cavity is possible.

The advantage of the pressuriser device 1 of the present invention is that pressure can be maintained on the cement from introduction of the cement through to cure without any disturbance from the pressuriser.

Improvements and modifications can be made to the aforesaid without departing from the present invention.

## Claims

1. A pressuriser device (1) having a collar (4) defining an opening (14) for accommodating a prosthetic component, and a hood (3) attached to the collar (4) which can enclose at least a portion of the central opening (14) of the collar,
**characterised in that**
the hood (3) is moveable between two positions, a first position in which the hood covers the opening (14) and a second position in which the opening (14) is uncovered.

2. A pressuriser device (1) as claimed in claim 1 wherein the collar (4) has an outer surface corresponding to the shape of a bone cavity in which the prosthetic component is to be inserted.

3. A pressuriser device (1) as claimed in claim 1 or claim 2 wherein the hood (3) has a hoop (20) extending across the opening in the collar

4. A pressuriser device (1) as claimed in claim 3 wherein the hoop (20) conforms to the shape of the prosthetic component.

5. A pressuriser device (1) as claimed in claim 3 or claim 4 wherein the hoop (20) is attached to the collar (4) by a sprung hinge (22) against which it can fold laterally.

6. A pressuriser device (1) as claimed in any of claims 3 to 5 wherein a body of the hood (3) extends from the hoop to the collar.

7. A pressuriser device (1) as claimed in any of the preceding claims wherein the body of the hood (3) corresponds to the shape of the prosthetic component to be inserted.

8. A pressuriser device (1) as claimed in any of the preceding claims wherein the hood (3) is attached to the collar (4) along the anterior and posterior aspects of the collar (4).

9. A pressuriser device (1) as claimed in any of the preceding claims wherein the hood (3) is attached by a tearable and flexible membrane.

10. A pressuriser device (1) as claimed in any of the preceding claims wherein the hood (3) is lined with an insert (24) to stiffen the hood.

11. A pressuriser device (1) as claimed in claim 10 wherein the insert (24) conforms to the shape of the prosthetic component.

12. A pressuriser device (1) substantially as hereinbefore described with reference to the accompanying drawings.

## Patentansprüche

1. Druckhaltevorrichtung (1), die einen Kragen (4), der eine Öffnung (14) definiert, um eine prothetische Komponente aufzunehmen, und eine Haube (3) aufweist, die an dem Kragen (4) befestigt ist, die zumindest einen Abschnitt der zentralen Öffnung (14) des Kragens umschließen kann, **dadurch gekennzeichnet, dass**
die Haube (3) zwischen zwei Positionen bewegbar ist, einer ersten Position, in der die Haube die Öffnung (14) bedeckt, und einer zweiten Position, in der die Öffnung (14) unbedeckt ist.

2. Druckhaltevorrichtung (1) nach Anspruch 1, **dadurch gekennzeichnet, dass**
der Kragen (4) eine äußere Oberfläche aufweist, die der Form eines Knochenhohlraums entspricht, in den die prothetische Komponente einzusetzen ist.

3. Druckhaltevorrichtung (1) nach Anspruch 1 oder Anspruch 2, **dadurch gekennzeichnet, dass**
die Haube (3) einen Reifen (20) aufweist, der sich über die Öffnung in dem Kragen erstreckt.

4. Druckhaltevorrichtung (1) gemäß Anspruch 3, **dadurch gekennzeichnet, dass**
der Reifen (20) sich an die Form der prothetischen Komponente anpasst.

5. Druckhaltevorrichtung (1) nach Anspruch 3 oder Anspruch 4, **dadurch gekennzeichnet, dass**
der Reifen (20) an dem Kragen (4) durch ein Federgelenk (22) befestigt ist, gegen das er sich seitwärts falten kann.

6. Druckhaltevorrichtung (1) gemäß einem der Ansprüche 3 bis 5, **dadurch gekennzeichnet, dass**
ein Körper der Haube (3) sich von dem Reifen zu dem Kragen erstreckt.

7. Druckhaltevorrichtung (1) gemäß einem der vorangehenden Ansprüche, **dadurch gekennzeichnet, dass**
der Körper der Haube (3) der Form der einzusetzenden prothetischen Komponente entspricht.

8. Druckhaltevorrichtung (1) gemäß einem der vorangehenden Ansprüche, **dadurch gekennzeichnet, dass**
die Haube (3) an dem Kragen (4) entlang den vorderen und hinteren Seiten des Kragens (4) befestigt ist.

9. Druckhaltevorrichtung (1) nach einem der vorangehenden Ansprüche, **dadurch gekennzeichnet, dass**
die Haube (3) durch eine zerreißbare und flexible Membran befestigt ist.

10. Druckhaltevorrichtung (1) gemäß einem der vorangehenden Ansprüche, **dadurch gekennzeichnet, dass**
die Haube (3) mit einem Einsatz (24) ausgekleidet ist, um die Haube zu versteifen.

11. Druckhaltevorrichtung (1) nach Anspruch 10, **dadurch gekennzeichnet, dass**
der Einsatz (24) sich an die Form der prothetischen Komponente anpasst.

12. Druckhaltevorrichtung (1) im wesentlichen wie oben beschrieben mit Referenz zu den beiliegenden Zeichnungen.

## Revendications

1. Dispositif de mise sous pression (1) comprenant un collet (4) définissant une ouverture (14) permettant de recevoir un composant prothétique, et un capuchon (3) fixé au collet (4) et pouvant recouvrir une partie au moins de l'ouverture centrale (14) du collet, **caractérisé en ce que** le capuchon (3) peut se déplacer entre deux positions, à savoir une première position dans laquelle le capuchon couvre l'ouverture (14) et une seconde position dans laquelle l'ouverture (14) est découverte.

2. Dispositif de mise sous pression (1), tel que revendiqué dans la revendication 1, dans lequel le collet (4) possède une surface externe correspondant à la forme d'une cavité osseuse dans laquelle le composant prothétique doit être inséré.

3. Dispositif de mise sous pression (1), tel que revendiqué dans la revendication 1 ou la revendication 2, dans lequel le capuchon (3) comporte un arceau (20) s'étendant à travers l'ouverture du collet.

4. Dispositif de mise sous pression (1), tel que revendiqué dans la revendication 3, dans lequel l'arceau (20) s'adapte à la forme du composant prothétique.

5. Dispositif de mise sous pression (1), tel que revendiqué dans la revendication 3 ou la revendication 4, dans lequel l'arceau (20) est fixé au collet (4) par une charnière élastique (22) contre laquelle il peut être pressélatéralement.

6. Dispositif de mise sous pression (1), tel que revendiqué dans l'une quelconque des revendications 3 à 5, dans lequel un corps du capuchon (3) s'étend depuis l'arceau jusqu'au collet.

7. Dispositif de mise sous pression (1), tel que revendiqué dans l'une quelconque des revendications précédentes, dans lequel le corps du capuchon (3) correspond à la forme du composant prothétique devant être inséré.

8. Dispositif de mise sous pression (1), tel que revendiqué dans l'une quelconque des revendications précédentes, dans lequel le capuchon (3) est attaché au collet (4) le long des faces antérieure et postérieure du collet (4).

9. Dispositif de mise sous pression (1), tel que revendiqué dans l'une quelconque des revendications précédentes, dans lequel le capuchon (3) est attaché par une membrane déchirable et flexible.

10. Dispositif de mise sous pression (1), tel que revendiqué dans l'une quelconque des revendications précédentes, dans lequel le capuchon (3) est renforcé par un insert (24) de manière à le rigidifier.

11. Dispositif de mise sous pression (1), tel que revendiqué dans la revendication 10, dans lequel l'insert (24) s'adapte à la forme du composant prothétique.

12. Dispositif de mise sous pression (1) essentiellement tel que décrit précédemment dans le cadre des dessins d'accompagnement.
